# EUROPEAN PATENT SPECIFICATION

(11) **EP 3 090 417 B1**
(45) Date of publication and mention of the grant of the patent: **29.04.2020**
(21) Application number: 15751382.1
(22) Date of filing: 19.02.2015
(51) Int. Cl.: G01C 21/12, G06F 1/16, G06F 1/32, G06F 3/01, G08B 7/00, H04L 29/06, H04W 12/06

(54) **SMART WEARABLE DEVICES AND METHODS FOR ACQUISITION OF SENSORIAL INFORMATION FROM SMART DEVICES**
AM KÖRPER TRAGBARE INTELLIGENTE VORRICHTUNGEN ZUR ERFASSUNG VON SENSORISCHEN INFORMATIONEN VON INTELLIGENTEN VORRICHTUNGEN
DISPOSITIFS INTELLIGENTS VESTIMENTAIRES ET PROCÉDÉS D'ACQUISITION D'INFORMATIONS SENSORIELLES À PARTIR DE DISPOSITIFS INTELLIGENTS

(30) Priority: 24.02.2014 US 201461943837 P
(43) Date of publication of application: 09.11.2016
(73) Proprietor: Sony Corporation, Tokyo 108-0075 (JP); Sony Corporation of America, New York, NY 10022 (US)
(72) Inventor: TANAKA, Nobuo, Park Ridge, New Jersey 07656 (US); ELGORT, Vladimir, Park Ridge, New Jersey 07656 (US); DANIELSON, Jacelyn, San Mateo, California 94404 (US); KALACHEV, Anton, San Mateo, California 94404 (US); WONG, John, Morristown, New Jersey 07960 (US); DACOSTA, Behram, San Jose, California 95112-4508 (US); BHAT, Udupi Ramanath, San Jose, California 95112-4508 (US); COPERE, Ludovic, San Jose, California 95112-4508 (US); KATAOKA, Masaki, Tokyo 108-0075 (JP)
(74) Representative: D Young & Co LLP
(86) International application number: PCT/US2015/016606
(87) International publication number: WO 2015/127070

(56) References cited:
- US-A1- 2011 018 731
- US-A1- 2012 316 455
- US-A1- 2013 135 108
- US-A1- 2013 217 978
- US-A1- 2013 290 427

## Description

### BACKGROUND

### 1. Field of the Technology

This technology pertains generally to smart wearable devices and more particularly to smart devices capable of acquiring sensor data from multiple sources.

### 2. Discussion

Multiple sensors, associated with smart wearable devices, with capabilities to collect data by acquiring biological or physical data of a user of a wearable device (heart rate, blood oxygen and sugar levels, body temperature, etc.), capabilities for determining physical or mental status of the user, the location of the user, and capabilities for collecting data on the environment (location, altitude, air pollution, distance traveled, external temperature, etc.), are often concentrated in the same location or engaged in the same activity. However, these sensors may be located on different users or different locations of the same wearer. Therefore, it is desirable for a smart wearable device to have the capability to acquire needed data from different devices.

US 2012/0316455 A1 discloses a wearable sensor device having a communications facility configured to transfer data between the wearable device and another device during an activity.

### BRIEF SUMMARY

This disclosure describes embodiments of smart wearable devices and methods for acquiring sensorial data from surrounding smart wearable devices and non-wearable devices. This data may be used to fill in the gaps when a wearable smart device collects incomplete or inaccurate data from sensors configured to, for example, collect biological data about the user or environmental and contextual data about an activity the user is engaged in.

In an example embodiment, a single smart wearable device user from a group of users or the smart wearable device itself may require additional information to improve accuracy of the data collected by its sensors or may require additional information to augment data collected about an experience or the contextual environment or to adjust the behavior of the smart wearable device based on the information collected by the surrounding sensors.

An exemplary embodiment of the method provides the capability to discover missing sensor information collected by a group of smart wearable device sensors engaged in the same activity in order to recreate an environment or experience based on defined criteria, where each smart wearable device sensor has its own contribution to make to the total data set. Additionally, a signal may be triggered in response to collected sensor data to activate a function on other wearable or non-wearable devices or change the logic for data collection.

Further aspects of the technology will be brought out in the following portions of the specification, wherein the detailed description is for the purpose of fully disclosing preferred embodiments of the technology without placing limitations thereon.

The present invention is defined by the appended claims. Particular embodiments are set out in the respective dependent claims. Embodiments which do not fall within the scope of the claims do not describe part of the present invention.

### BRIEF DESCRIPTION OF THE SEVERAL VIEWS OF THE DRAWING(S)

The technology described herein will be more fully understood by reference to the following drawings which are for illustrative purposes only:
FIG. 1 is a schematic diagram of an embodiment of a smart wearable network described herein.
FIG. 2 is a functional block diagram of an embodiment of a smart wearable device described herein.
FIG. 3 is a schematic diagram of an embodiment of a smart wearable device requesting and receiving sensorial data from surrounding smart devices.
FIG. 4 is a flow diagram of an exemplary method for requesting and receiving sensorial data from surrounding smart devices.

### DETAILED DESCRIPTION

### A. Generalized System Implementation.

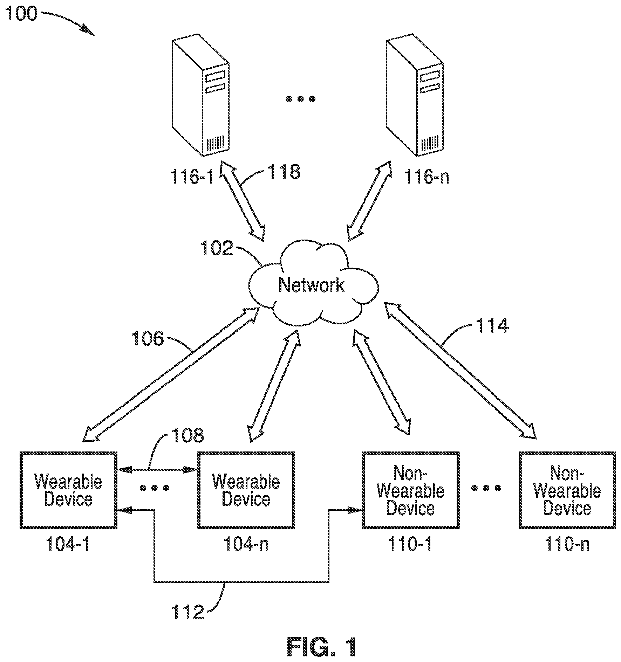

FIG. 1 illustrates a generalized networked infrastructure (e.g., system) 100 that includes a network 102 such as the Internet. One or more smart wearable devices 104-1 through 104-n described herein may be enabled to communicate with each other and/or with the network by means of wired or wireless connections. Further, any of the smart wearable devices may be enabled to communicate with the network by means of wired or wireless connections. Smart wearable devices according to this disclosure are described below.

One or more smart wearable devices 104-1 through 104-n also may be enabled to communicate with one or more non-wearable devices 106-1 through 106-n by means of wired or wireless connections. The non-wearable devices may be any conventional "smart" device with a processor, associated operating system, and communications interface. Examples of non-wearable devices include smartphones, tablet computers, laptop computers, desktop computers, and set top boxes.

One or more servers 108-1 through 108-n may be provided in a client-server configuration and connected to the network by means of wired or wireless connections. The servers may include standalone servers, cluster servers, networked servers, or servers connected in an array to function like a large computer.

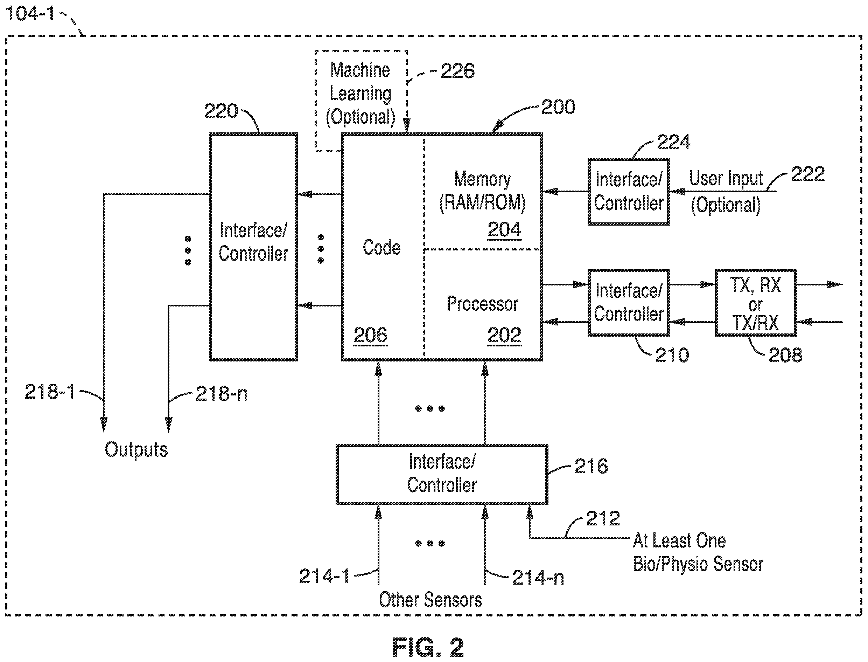

FIG. 2 illustrates a generalized embodiment of a smart wearable device according to this disclosure. It will be appreciated that the embodiment shown may be modified or customized to enable performing the functions described herein. In the exemplary embodiment shown, the smart wearable device includes an "engine" 200 having a processor 202, memory 204, and application software code 206. The processor 202 can be any suitable conventional processor. The memory 204 may include any suitable conventional RAM type memory and/or ROM type memory with associated storage space for storing the application programming code 206.

A conventional wired or wireless communications module 208 (e.g., transmitter or receiver or transceiver) may be included as needed for performing one or more of the functions of the smart wearable device described herein. Examples of wireless communication capabilities that can be provided include, but are not limited to, Bluetooth, WiFi, infrared, cellular, and near field communication. One or more conventional interfaces or controllers 210 may also be provided if needed. Examples of interfaces or controllers include, but are not limited to, analog to digital converters, digital to analog converters, buffers, etc.

The device may include at least one input 212 for a biological or physiological sensor for providing input to the device to perform one or more of the functions described herein. Sensor inputs 214-1 through 214-n for optional sensors may be included as well. These optional input sensors may include, but are not limited to, accelerometers, temperature sensors, altitude sensors, motion sensors, position sensors, and other sensors to perform the function(s) described herein. One or more conventional interfaces or controllers 216 may be provided if needed for the sensors. Examples of interfaces or controllers include, but are not limited to, analog to digital converters, digital to analog converters, buffers, etc.

Additionally, the device may include one or more outputs 218-1 through 218-n to drive one or more output devices (and include those output devices). These output devices may include, but are not limited to, haptic output devices, telemetry devices, visual devices, and audible devices. One or more conventional interfaces or controllers 220 may be provided if needed for the output devices. Examples of interfaces or controllers include, but are not limited to, analog to digital converters, digital to analog converters, buffers, etc.

A user input 222 may be provided according to the functions described herein. The user input may, for example, initiate one or more functions, terminate one or more functions, or intervene in a running process. The user input can be any conventional input device, including but not limited to, manual switches, touch sensors, magnetic sensors, proximity sensors, etc. One or more conventional interfaces or controllers 224 may be provided if needed for the output devices. Examples of interfaces or controllers include, but are not limited to, analog to digital converters, digital to analog converters, buffers, etc.

Depending on the function(s) described herein, the engine 200 may also include a feedback loop 226 for machine learning or other functions.

It will be appreciated that a smart wearable device as described herein would necessarily include a housing or carrier for the above-described components. It will further be appreciated that, as used herein, the term "smart wearable device" means a device that would be worn or otherwise associated with the body of a user and be "connected" to the user by means of at least one sensor for sensing one or more biological or physiological conditions of the user.

The particular form of the housing or carrier (i.e., wearable platform) can vary according to choice and suitability for performing the functions described herein. Examples of wearable platforms include, but are not limited to, wrist worn devices, head worn devices, arm worn devices, leg worn devices, ankle worn devices, foot worn devices, watches, eyeglasses, rings, bracelets, necklaces, articles of jewelry, articles of clothing, shoes, hats, contact lenses, etc.

It will further be appreciated that the input sensors and output devices may be integrated into the wearable platform, or may be external to the wearable platform, as is desired and/or suitable for the function(s) of the smart wearable device.

### B. Smart Wearable Device for Acquisition of Sensorial Information from Surrounding Smart Devices.

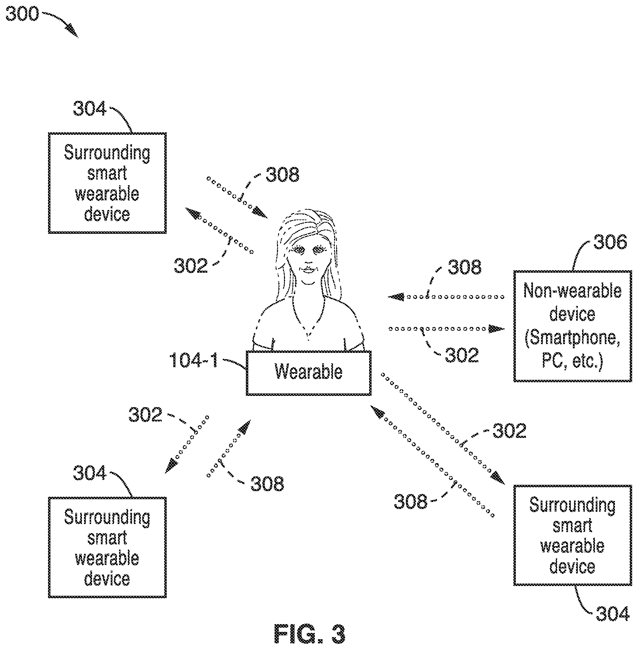

The disclosed devices and methods may facilitate the acquisition of accurate, missing or functional data from smart devices within proximity to a smart wearable device. Referring now to FIG. 3, a schematic diagram 300 of an embodiment of a smart wearable device 104-1 (see also FIG. 2) is shown. In this embodiment, the smart wearable device may broadcast a request 302 to surrounding smart devices, which may include for example, other smart wearable devices 304 and non-wearable smart devices 306. The broadcast request can allow the smart wearable device to determine whether there are any surrounding smart devices that have sensors which may be acquiring data 308 that is relevant to the data in which the smart wearable device 104-1 is acquiring with its own sensors. This broadcast request can also determine the accessibility of the senor data on these surrounding smart devices 304, 306 so that the smart wearable device 104-1 can acquire the relevant sensor data. Although not limited to the following applications, the sensor data acquired 308 by the surrounding smart devices 304, 306 may be used by the smart wearable device 104-1 to improve the accuracy of the data collected by the device's own sensors, to augment the data collected by the device's own sensors, or to adjust the functions being performed by the wearable smart device 104-1. The smart wearable device may determine and filter out data that is not relevant to the current activity or function (i.e. sensor data) of the wearable smart device based on given criteria. Alternatively, the surrounding smart devices 304, 306 can analyze the sensor data being requested for relevancy before the sensor data is transferred to the smart wearable device 104-1 that requested it.

The smart wearable device 104-1 may also use the sensor data retrieved by the surrounding smart devices to make a decision to change the behavior of the smart wearable device 104-1 or enable an additional function on the smart wearable device 104-1 that may be useful in performing an activity. Conversely, the smart wearable device may also use the sensor data retrieved by the surrounding smart devices to make a decision to send a signal to one or more of the surrounding devices 304, 306 or to trigger activation or deactivation of a function. Examples of this include taking a picture, enabling higher sampling rates of data collection, etc.

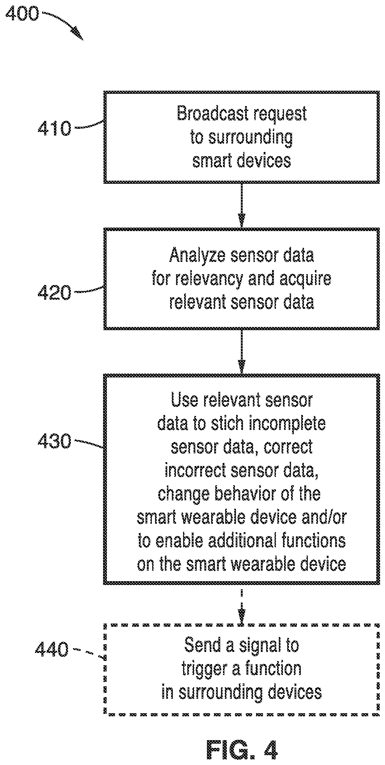

Referring now to FIG. 4, a flow diagram 400 is shown of an embodiment of a smart wearable device requesting and receiving sensorial data from surrounding smart devices. In this embodiment, the smart wearable device may first broadcast a request to any surrounding smart devices 410. The broadcasted request may allow the smart wearable device to determine sensors available in the proximity, sensors engaged in the same activity, or type of the sensor data available as well as relevancy of the sensor data. If requested or relevant sensor data is available on any of the surrounding devices, a request may be sent to acquire relevant portions of the data from one or multiple wearable or non-wearable smart devices, determined by a given criteria 420. The sensor data can be analyzed for relevancy by the device requesting the sensor data or analyzed by the device transferring the sensor data.

Acquired data may be processed by the sensor requesting the data and used to improve the accuracy of the sensor data collected or used to make a decision to change the behavior of the smart wearable device, or to enable additional functions on the smart wearable device engaged in an activity 430. Implementation of an embodiment of the method may allow the smart wearable device to send a request to the surrounding devices to trigger a function (e.g. taking a picture, enabling higher sampling rate of data collection, etc.) 440.

In one embodiment, a user may be wearing two devices. The two devices, for example, may be a smart wearable device with an accelerometer watch and another smart wearable device with a heart rate monitor (HRM) and a GPS. The accelerometer watch may broadcast to the HRM in order to receive metadata associated with sensory input captured by HRM. The accelerometer watch can then check the metadata and identify GPS sensor data as relevant or not relevant to its own data that's been captured. This may be carried out by organizing the metadata into a data structure having several fields. One field can list a unique identifier and another field associated with this unique identifier can describe a property (e.g. a rotational position, a linear distance, etc.) or perhaps measurement units (e.g. degrees, feet, etc.). The processor may check the sensory metadata associated with its sensors and look for matching fields of property (or measurement units, etc.). Matches can then be flagged as "relevant sensor data."

The processor of the accelerometer watch can correct its position data acquired from the accelerometer by comparing it to the GPS position data from the HRM. The two position data sets may be averaged with the final position data set displayed to the user or communicated from the accelerometer watch to another device via an application or other software. Alternatively, if the GPS position data is incomplete (some position data is missing due to line of sight blockage with a satellite providing GPS data, or the GPS receiver is turned off to save power, either intentionally or by power saving mode of the HRM), then missing data from the accelerometer watch can be substituted. In addition, when the accelerometer watch is acquiring sensor data from another device, in this case the HRM with the GPS, the accelerometer watch can make a further determination of which of the relevant sensor data is optimal for its purposes: (i) use only GPS because it's more accurate; (ii) use only the accelerometer to save battery power because power remaining in watch is below a predetermined threshold; (iii) use both; (iv) use neither. The choices, (i) through (iv), can be performed automatically or in response to user direction.

In another embodiment using this example of the watch accelerometer and the HRM/GPS, the two devices may capture similar biological sensor data, such as the wearer's pulse, but from different body locations (the watch from the user's wrist; the HRM from chest strap). The watch can compare the pulse data from its sensors to the sensor data acquired from the HRM. Based on the wearer's settings and internal analysis, the watch records pulse data from the chest unless such data is missing, in which case the wrist pulse data is substituted to provide a complete data set of the wearer's pulse. The wearer's settings can establish the wearer's preference to use chest pulse data as the wearer believes such data (compared to wrist collected pulse data) is more accurate for aerobic exercise.

Embodiments of the present technology may be described with reference to flowchart illustrations of methods and systems according to embodiments of the technology, and/or algorithms, formulae, or other computational depictions, which may also be implemented as computer program products. In this regard, each block or step of a flowchart, and combinations of blocks (and/or steps) in a flowchart, algorithm, formula, or computational depiction can be implemented by various means, such as hardware, firmware, and/or software including one or more computer program instructions embodied in computer-readable program code logic. As will be appreciated, any such computer program instructions may be loaded onto a computer, including without limitation a general purpose computer or special purpose computer, or other programmable processing apparatus to produce a machine, such that the computer program instructions which execute on the computer or other programmable processing apparatus create means for implementing the functions specified in the block(s) of the flowchart(s).

Accordingly, blocks of the flowcharts, algorithms, formulae, or computational depictions support combinations of means for performing the specified functions, combinations of steps for performing the specified functions, and computer program instructions, such as embodied in computer-readable program code logic means, for performing the specified functions. It will also be understood that each block of the flowchart illustrations, algorithms, formulae, or computational depictions and combinations thereof described herein, can be implemented by special purpose hardware-based computer systems which perform the specified functions or steps, or combinations of special purpose hardware and computer-readable program code logic means.

Furthermore, these computer program instructions, such as embodied in computer-readable program code logic, may also be stored in a computer-readable memory that can direct a computer or other programmable processing apparatus to function in a particular manner, such that the instructions stored in the computer-readable memory produce an article of manufacture including instruction means which implement the function specified in the block(s) of the flowchart(s). The computer program instructions may also be loaded onto a computer or other programmable processing apparatus to cause a series of operational steps to be performed on the computer or other programmable processing apparatus to produce a computer-implemented process such that the instructions which execute on the computer or other programmable processing apparatus provide steps for implementing the functions specified in the block(s) of the flowchart(s), algorithm(s), formula(e), or computational depiction(s).

It will further be appreciated that "programming" as used herein refers to one or more instructions that can be executed by a processor to perform a function as described herein. The programming can be embodied in software, in firmware, or in a combination of software and firmware. The programming can be stored local to the device in non-transitory media, or can be stored remotely such as on a server, or all or a portion of the programming can be stored locally and remotely. Programming stored remotely can be downloaded (pushed) to the device by user initiation, or automatically based on one or more factors, such as, for example, location, a timing event, detection of an object, detection of a facial expression, detection of location, detection of a change in location, or other factors. It will further be appreciated that as used herein, that the terms processor, central processing unit (CPU), and computer are used synonymously to denote a device capable of executing the programming and communication with input/output interfaces and/or peripheral devices.

Although the description above contains many details, these should not be construed as limiting the scope of the technology but as merely providing illustrations of some of the presently preferred embodiments of this technology. Therefore, it will be appreciated that the scope of the present technology fully encompasses other embodiments which may become obvious to those skilled in the art, and that the scope of the present technology is accordingly to be limited by nothing other than the appended claims, in which reference to an element in the singular is not intended to mean "one and only one" unless explicitly so stated, but rather "one or more." All structural, chemical, and functional equivalents to the elements of the above-described preferred embodiment that are known to those of ordinary skill in the art are expressly incorporated herein by reference and are intended to be encompassed by the present claims. Moreover, it is not necessary for a device or method to address each and every problem sought to be solved by the present technology, for it to be encompassed by the present claims. Furthermore, no element, component, or method step in the present disclosure is intended to be dedicated to the public regardless of whether the element, component, or method step is explicitly recited in the claims.

## Claims

1. A smart wearable device (104-1), the device comprising:
(a) a housing, wherein the housing encases components of a smart wearable device;
(b) one or more sensors configured to acquire sensor data, wherein at least one sensor is a biological sensor configured to acquire biological data about a user;
(c) one or more communications interfaces (208);
(d) a processor (202); and
(e) programming residing in a non-transitory computer readable medium, wherein the programming is executable by the computer processor and configured to:
(i) broadcast a request (302) to surrounding smart devices (304, 306) to determine availability of accessible surrounding smart devices engaged in acquiring sensor data which is relevant to the sensor data acquired by the one or more sensors of the smart wearable device, wherein relevancy is based on defined criteria; and
(ii) acquire the relevant sensor data (308) from accessible surrounding smart devices, wherein the relevant sensor data is used to substitute sensor data acquired by the one or more sensors of the smart wearable device that is incomplete or to correct incorrect sensor data acquired by the one or more sensors of the smart wearable device on the smart wearable device.

2. The device of claim 1, wherein at least one of the one or more sensors is an environmental sensor configured to acquire contextual sensor data.

3. The device of claim 1, wherein said programming is further configured to:
send a signal to one or more of the surrounding smart devices to trigger activation or deactivation of a function or change the logic for acquisition of sensor data by the additional smart wearable devices.

4. The device of claim 1, wherein the smart wearable device has a platform selected from the group consisting of hand worn devices, finger worn devices, wrist worn devices, head worn devices, arm worn devices, leg worn devices, ankle worn devices, foot worn devices, toe worn devices, watches, eyeglasses, rings, bracelets, necklaces, articles of jewelry, articles of clothing, shoes, hats, contact lenses, and gloves.

5. The device of claim 1, wherein the one or more communications interfaces are selected from the group consisting of a wired communications interface, a wireless communications interface, a cellular communications interface, a WiFi communications interface, a near field communications interface, an infrared communications interface, and a Bluetooth communications interface.

6. A computer implemented method for acquiring sensorial data from surrounding smart devices, the method comprising:
(a) providing a smart wearable device, wherein the smart wearable device comprises:
(i) a housing, wherein the housing encases components of a smart wearable device;
(ii) one or more sensors configured to acquire sensor data, wherein at least one sensor is a biological sensor configured to acquire biological data about a user;
(iii) one or more communications interfaces; and
(iv) a processor;
(b) broadcasting a request from the smart wearable device to surrounding smart devices to determine availability of accessible surrounding smart devices engaged in acquiring sensor data which is relevant to the sensor data acquired by the one or more sensors of the smart wearable device, wherein relevancy is based on defined criteria; and
(c) acquiring the relevant sensor data from accessible surrounding smart devices, wherein the relevant sensor data is used to substitute sensor data acquired by the one or more sensors of the smart wearable device that is incomplete or to correct incorrect sensor data acquired by the one or more sensors of the smart wearable device on the smart wearable device;
(d) wherein said method is performed by executing programming on at least one computer processor, said programming residing on a non-transitory medium readable by the computer processor.

7. The method of claim 6, wherein the at least one of the one or more sensors is an environmental sensor configured to acquire contextual sensor data.

8. The method of claim 6, further comprising:
sending a signal to one or more of the surrounding smart devices to trigger activation or deactivation of a function or change the logic for acquisition of sensor data by the additional smart wearable devices.

9. The method of claim 6, wherein the smart wearable device has a platform selected from the group consisting of hand worn devices, finger worn devices, wrist worn devices, head worn devices, arm worn devices, leg worn devices, ankle worn devices, foot worn devices, toe worn devices, watches, eyeglasses, rings, bracelets, necklaces, articles of jewelry, articles of clothing, shoes, hats, contact lenses, and gloves.

10. The method of claim 6, wherein the one or more communications interfaces are selected from the group consisting of a wired communications interface, a wireless communications interface, a cellular communications interface, a WiFi communications interface, a near field communications interface, an infrared communications interface, and a Bluetooth communications interface.

11. A system for acquiring sensorial data from surrounding smart devices, the system comprising:
(a) one or more surrounding smart devices, the one or more smart device comprising:
(i) a housing, wherein the housing encases components of a smart wearable device;
(ii) one or more sensors, wherein at least one sensor is configured to acquire biological data;
(iii) one or more communications interfaces;
(iv) a processor; and
(v) programming residing in a non-transitory computer readable medium, wherein the programming is executable by the computer processor and configured to:
receive a request for sensor data from a smart wearable device; and
transmit the requested sensor data to the smart wearable device; and
(b) a smart wearable device comprising:
(i) a housing, wherein the housing encases components of a smart wearable device;
(ii) one or more sensors configured to acquire sensor data, wherein at least one sensor is a biological sensor configured to acquire biological data about a user;
(iii) one or more communications interfaces;
(iv) a processor; and
(v) programming residing in a non-transitory computer readable medium, wherein the programming is executable by the computer processor and configured to:
broadcast a request to the surrounding smart devices to determine availability of accessible surrounding smart devices engaged in acquiring sensor data which is relevant to the sensor data acquired by the one or more sensors of the smart wearable device, wherein relevancy is based on defined criteria; and
acquire the relevant sensor data from the accessible surrounding smart devices, wherein the relevant sensor data is used to substitute sensor data acquired by the one or more sensors of the smart wearable device that is incomplete or to correct incorrect sensor data acquired by the one or more sensors of the smart wearable device on the smart wearable device.

12. The system of claim 11, wherein at least one of the one or more sensors of the surrounding smart devices is an environmental sensor configured to acquire contextual sensor data, and
at least one of the one or more sensors of the smart wearable device is an environmental sensor configured to acquire contextual sensor data.

13. The system of claim 11, wherein said programming on the wearable smart device is further configured to:
send a signal to one or more of the surrounding smart devices to trigger activation or deactivation of a function or change the logic for acquisition of sensor data by the additional smart wearable devices.

14. The system of claim 11, wherein the smart wearable device has a platform selected from the group consisting of hand worn devices, finger worn devices, wrist worn devices, head worn devices, arm worn devices, leg worn devices, ankle worn devices, foot worn devices, toe worn devices, watches, eyeglasses, rings, bracelets, necklaces, articles of jewelry, articles of clothing, shoes, hats, contact lenses, and gloves.

15. The system of claim 11, wherein the one or more communications interfaces on the smart wearable device are selected from the group consisting of a wired communications interface, a wireless communications interface, a cellular communications interface, a WiFi communications interface, a near field communications interface, an infrared communications interface, and a Bluetooth communications interface.

## Patentansprüche

1. Am Körper tragbare intelligente Vorrichtung (104-1), wobei die Vorrichtung folgendes umfasst:
(a) ein Gehäuse, wobei das Gehäuse Komponenten einer intelligenten am Körper tragbaren Vorrichtung umschließt;
(b) einen oder mehrere Sensoren, die ausgelegt sind, um Sensordaten zu erfassen, wobei mindestens ein Sensor ein biologischer Sensor ist, der ausgelegt ist, um biologische Daten eines Anwenders zu erfassen;
(c) eine oder mehrere Kommunikationsschnittstellen (208) ;
(d) einen Prozessor (202); und
(e) Programmierung, die in einem nicht-flüchtigen computerlesbaren Medium liegt, wobei die Programmierung durch den Computerprozessor ausführbar ist und ausgelegt ist, um folgendes durchzuführen:
(i) Verbreiten einer Anfrage (302) an umgebende intelligente Vorrichtungen (304, 306) mittels Broadcast, um die Verfügbarkeit von zugänglichen umgebenden intelligenten Vorrichtungen zu ermitteln, die mit der Erfassung von Sensordaten beschäftigt sind, die für die Sensordaten relevant sind, die durch den einen oder die mehreren Sensoren der intelligenten am Körper tragbaren Vorrichtung erfasst werden, wobei Relevanz auf definierten Kriterien basiert; und
(ii) Erfassen der relevanten Sensordaten (308) von zugänglichen umgebenden intelligenten Vorrichtungen, wobei die relevanten Sensordaten verwendet werden, um Sensordaten zu ersetzen, die durch den einen oder die mehreren Sensoren der intelligenten am Körper tragbaren Vorrichtung erfasst werden, die unvollständig sind, oder um fehlerhafte Sensordaten zu korrigieren, die durch den einen oder die mehreren Sensoren der intelligenten am Körper tragbaren Vorrichtung auf der intelligenten am Körper tragbaren Vorrichtung erfasst wurden.

2. Vorrichtung nach Anspruch 1, wobei mindestens einer von dem einen oder den mehreren Sensoren ein Umgebungssensor ist, der ausgelegt ist, um Kontextsensordaten zu erfassen.

3. Vorrichtung nach Anspruch 1, wobei die Programmierung des Weiteren ausgelegt ist, um folgendes durchzuführen:
Senden eines Signals an eine oder mehrere der umgebenden intelligenten Vorrichtungen, um Aktivierung oder Deaktivierung einer Funktion auszulösen oder die Logik zur Erfassung von Sensordaten durch die zusätzlichen intelligenten am Körper tragbaren Vorrichtungen zu verändern.

4. Vorrichtung nach Anspruch 1, wobei die intelligente am Körper tragbare Vorrichtung eine Plattform aufweist, die ausgewählt ist aus der Gruppe bestehend aus an der Hand getragenen Vorrichtungen, am Finger getragenen Vorrichtungen, am Handgelenk getragenen Vorrichtungen, am Kopf getragenen Vorrichtungen, am Arm getragenen Vorrichtungen, am Bein getragenen Vorrichtungen, am Knöchel getragenen Vorrichtungen, am Fuß getragenen Vorrichtungen, am Zeh getragenen Vorrichtungen, Armbanduhren, Brillengläsern, Ringen, Armbändern, Halsketten, Schmuckgegenständen, Bekleidungsartikeln, Schuhen, Kopfbedeckungen, Kontaktlinsen und Handschuhen.

5. Vorrichtung nach Anspruch 1, wobei die eine oder mehreren Kommunikationsschnittstellen ausgewählt ist bzw. sind aus der Gruppe bestehend aus einer verdrahteten Kommunikationsschnittstelle, einer drahtlosen Kommunikationsschnittstelle, einer Mobilfunkkommunikationsschnittstelle, einer WiFi-Kommunikationsschnittstelle, einer Nahfeldkommunikationsschnittstelle, einer Infrarotkommunikationsschnittstelle und einer Bluetooth-Kommunikationsschnittstelle.

6. Computerimplementiertes Verfahren zum Erfassen von sensorbezogenen Daten aus umgebenden intelligenten Vorrichtungen, wobei das Verfahren umfasst:
(a) Bereitstellen einer intelligenten am Körper tragbaren Vorrichtung, wobei die intelligente am Körper tragbare Vorrichtung umfasst:
(i) ein Gehäuse, wobei das Gehäuse Komponenten einer intelligenten am Körper tragbaren Vorrichtung umschließt;
(ii) einen oder mehrere Sensoren, die ausgelegt sind, um Sensordaten zu erfassen, wobei mindestens ein Sensor ein biologischer Sensor ist, der ausgelegt ist, um biologische Daten eines Anwenders zu erfassen;
(iii) eine oder mehrere Kommunikationsschnittstellen; und
(iv) einen Prozessor;
(b) Verbreiten einer Anfrage von der intelligenten am Körper tragbaren Vorrichtung an umgebende intelligente Vorrichtungen mittels Broadcast, um die Verfügbarkeit von zugänglichen umgebenden intelligenten Vorrichtungen zu ermitteln, die mit der Erfassung von Sensordaten beschäftigt sind, die für die Sensordaten relevant sind, die durch den einen oder die mehreren Sensoren der intelligenten am Körper tragbaren Vorrichtung erfasst werden, wobei Relevanz auf definierten Kriterien basiert; und
(c) Erfassen der relevanten Sensordaten von zugänglichen umgebenden intelligenten Vorrichtungen, wobei die relevanten Sensordaten verwendet werden, um Sensordaten zu ersetzen, die durch den einen oder die mehreren Sensoren der intelligenten am Körper tragbaren Vorrichtung erfasst werden, die unvollständig sind, oder um fehlerhafte Sensordaten zu korrigieren, die durch den einen oder die mehreren Sensoren der intelligenten am Körper tragbaren Vorrichtung auf der intelligenten am Körper tragbaren Vorrichtung erfasst wurden;
(d) wobei das Verfahren durchgeführt wird, indem Programmierung auf mindestens einem Computerprozessor durchgeführt wird, wobei die Programmierung auf einem nicht-flüchtigen Medium liegt, das durch den Computerprozessor lesbar ist.

7. Verfahren nach Anspruch 6, wobei der mindestens eine von dem einen oder den mehreren Sensoren ein Umgebungssensor ist, der ausgelegt ist, um Kontextsensordaten zu erfassen.

8. Verfahren nach Anspruch 6, des Weiteren umfassend:
Senden eines Signals an eine oder mehrere der umgebenden intelligenten Vorrichtungen, um Aktivierung oder Deaktivierung einer Funktion auszulösen oder die Logik zur Erfassung von Sensordaten durch die zusätzlichen intelligenten am Körper tragbaren Vorrichtungen zu verändern.

9. Verfahren nach Anspruch 6, wobei die intelligente am Körper tragbare Vorrichtung eine Plattform aufweist, die ausgewählt ist aus der Gruppe bestehend aus an der Hand getragenen Vorrichtungen, am Finger getragenen Vorrichtungen, am Handgelenk getragenen Vorrichtungen, am Kopf getragenen Vorrichtungen, am Arm getragenen Vorrichtungen, am Bein getragenen Vorrichtungen, am Knöchel getragenen Vorrichtungen, am Fuß getragenen Vorrichtungen, am Zeh getragenen Vorrichtungen, Armbanduhren, Brillengläsern, Ringen, Armbändern, Halsketten, Schmuckgegenständen, Bekleidungsartikeln, Schuhen, Kopfbedeckungen, Kontaktlinsen und Handschuhen.

10. Verfahren nach Anspruch 6, wobei die eine oder mehreren Kommunikationsschnittstellen ausgewählt ist bzw. sind aus der Gruppe bestehend aus einer verdrahteten Kommunikationsschnittstelle, einer drahtlosen Kommunikationsschnittstelle, einer Mobilfunkkommunikationsschnittstelle, einer WiFi-Kommunikationsschnittstelle, einer Nahfeldkommunikationsschnittstelle, einer Infrarotkommunikationsschnittstelle und einer Bluetooth-Kommunikationsschnittstelle.

11. System zum Erfassen von sensorbezogenen Daten aus umgebenden intelligenten Vorrichtungen, wobei das System umfasst:
(a) eine oder mehrere umgebende intelligente Vorrichtungen, wobei die eine oder mehreren intelligenten Vorrichtungen umfasst bzw. umfassen:
(i) ein Gehäuse, wobei das Gehäuse Komponenten einer intelligenten am Körper tragbaren Vorrichtung umschließt;
(ii) einen oder mehrere Sensoren, wobei mindestens ein Sensor ausgelegt ist, um biologische Daten zu erfassen;
(iii) eine oder mehrere Kommunikationsschnittstellen;
(iv) einen Prozessor; und
(v) Programmierung, die in einem nicht-flüchtigen computerlesbaren Medium liegt, wobei die Programmierung durch den Computerprozessor ausführbar ist und ausgelegt ist, um folgendes durchzuführen:
Empfangen einer Anfrage nach Sensordaten von einer intelligenten am Körper tragbaren Vorrichtung; und
Übermitteln der angeforderten Sensordaten an die intelligente am Körper tragbare Vorrichtung; und
(b) eine intelligente am Körper tragbare Vorrichtung, welche folgendes umfasst:
(i) ein Gehäuse, wobei das Gehäuse Komponenten einer intelligenten am Körper tragbaren Vorrichtung umschließt;
(ii) einen oder mehrere Sensoren, die ausgelegt sind, um Sensordaten zu erfassen, wobei mindestens ein Sensor ein biologischer Sensor ist, der ausgelegt ist, um biologische Daten eines Anwenders zu erfassen;
(iii) eine oder mehrere Kommunikationsschnittstellen;
(iv) einen Prozessor; und
(v) Programmierung, die in einem nicht-flüchtigen computerlesbaren Medium liegt, wobei die Programmierung durch den Computerprozessor ausführbar ist und ausgelegt ist, um folgendes durchzuführen:
Verbreiten einer Anfrage an die umgebenden intelligenten Vorrichtungen mittels Broadcast, um die Verfügbarkeit von zugänglichen umgebenden intelligenten Vorrichtungen zu ermitteln, die mit der Erfassung von Sensordaten beschäftigt sind, die für die Sensordaten relevant sind, die durch den einen oder die mehreren Sensoren der intelligenten am Körper tragbaren Vorrichtung erfasst werden, wobei Relevanz auf definierten Kriterien basiert; und
Erfassen der relevanten Sensordaten von den zugänglichen umgebenden intelligenten Vorrichtungen, wobei die relevanten Sensordaten verwendet werden, um Sensordaten zu ersetzen, die durch den einen oder die mehreren Sensoren der intelligenten am Körper tragbaren Vorrichtung erfasst werden, die unvollständig sind, oder um fehlerhafte Sensordaten zu korrigieren, die durch den einen oder die mehreren Sensoren der intelligenten am Körper tragbaren Vorrichtung auf der intelligenten am Körper tragbaren Vorrichtung erfasst wurden.

12. System nach Anspruch 11, wobei mindestens einer von dem einen oder den mehreren Sensoren der umgebenden intelligenten Vorrichtungen ein Umgebungssensor ist, der ausgelegt ist, um Kontextsensordaten zu erfassen, und
mindestens einer von dem einen oder den mehreren Sensoren der intelligenten am Körper tragbaren Vorrichtung ein Umgebungssensor ist, der ausgelegt ist, um Kontextsensordaten zu erfassen.

13. System nach Anspruch 11, wobei die Programmierung der am Körper tragbaren intelligenten Vorrichtung des Weiteren ausgelegt ist, um folgendes durchzuführen:
Senden eines Signals an eine oder mehrere der umgebenden intelligenten Vorrichtungen, um Aktivierung oder Deaktivierung einer Funktion auszulösen oder die Logik zur Erfassung von Sensordaten durch die zusätzlichen intelligenten am Körper tragbaren Vorrichtungen zu verändern.

14. System nach Anspruch 11, wobei die intelligente am Körper tragbare Vorrichtung eine Plattform aufweist, die ausgewählt ist aus der Gruppe bestehend aus an der Hand getragenen Vorrichtungen, am Finger getragenen Vorrichtungen, am Handgelenk getragenen Vorrichtungen, am Kopf getragenen Vorrichtungen, am Arm getragenen Vorrichtungen, am Bein getragenen Vorrichtungen, am Knöchel getragenen Vorrichtungen, am Fuß getragenen Vorrichtungen, am Zeh getragenen Vorrichtungen, Armbanduhren, Brillengläsern, Ringen, Armbändern, Halsketten, Schmuckgegenständen, Bekleidungsartikeln, Schuhen, Kopfbedeckungen, Kontaktlinsen und Handschuhen.

15. System nach Anspruch 11, wobei die eine oder mehreren Kommunikationsschnittstellen an der intelligenten am Körper tragbaren Vorrichtung ausgewählt ist bzw. sind aus der Gruppe bestehend aus einer verdrahteten Kommunikationsschnittstelle, einer drahtlosen Kommunikationsschnittstelle, einer Mobilfunkkommunikationsschnittstelle, einer WiFi-Kommunikationsschnittstelle, einer Nahfeldkommunikationsschnittstelle, einer Infrarotkommunikationsschnittstelle und einer Bluetooth-Kommunikationsschnittstelle.

## Revendications

1. Dispositif portable intelligent (104-1), le dispositif comprenant :
(a) un boîtier, le boîtier renfermant des composants d'un dispositif portable intelligent ;
(b) un ou plusieurs capteurs configurés pour acquérir des données de capteur, au moins un capteur étant un capteur biologique configuré pour acquérir des données biologiques concernant un utilisateur ;
(c) une ou plusieurs interfaces de communication (208) ;
(d) un processeur (202) ; et
(e) une programmation se trouvant dans un support non transitoire lisible par ordinateur, la programmation étant exécutable par le processeur informatique et configurée pour :
(i) diffuser une requête (302) vers des dispositifs intelligents environnants (304, 306) afin de déterminer une disponibilité de dispositifs intelligents environnants accessibles engagés dans l'acquisition de données de capteur qui se rapportent aux données de capteur acquises par le ou les capteurs du dispositif portable intelligent, la pertinence se basant sur des critères prédéfinis ; et
(ii) acquérir les données de capteur (308) pertinentes à partir de dispositifs intelligents environnants accessibles, les données de capteur pertinentes servant à remplacer des données de capteur acquises par le ou les capteurs du dispositif portable intelligent qui sont incomplètes ou à corriger des données de capteur incorrectes acquises par le ou les capteurs du dispositif portable intelligent sur le dispositif portable intelligent.

2. Dispositif selon la revendication 1, dans lequel au moins un parmi le ou les capteurs est un capteur environnemental configuré pour acquérir des données de capteur contextuelles.

3. Dispositif selon la revendication 1, dans lequel ladite programmation est en outre configurée pour :
envoyer un signal à un ou plusieurs des dispositifs intelligents environnants afin de déclencher une activation ou une désactivation d'une fonction ou de changer la logique pour l'acquisition de données de capteur par les dispositifs portables intelligents supplémentaires.

4. Dispositif selon la revendication 1, dans lequel le dispositif portable intelligent possède une plateforme choisie dans le groupe consistant en dispositifs portés à la main, dispositifs portés au doigt, dispositifs portés au poignet, dispositifs portés sur la tête, dispositifs portés au bras, dispositifs portés à la jambe, dispositifs portés à la cheville, dispositifs portés au pied, dispositifs portés aux orteils, montres, lunettes, bagues, bracelets, colliers, articles de bijouterie, articles vestimentaires, chaussures, chapeaux, lentilles de contact et gants.

5. Dispositif selon la revendication 1, dans lequel la ou les interfaces de communication sont choisies dans le groupe consistant en une interface de communication câblée, une interface de communication sans fil, une interface de communication cellulaire, une interface de communication Wi-Fi, une interface de communication en champ proche, une interface de communication infrarouge et une interface de communication Bluetooth.

6. Procédé implémenté par ordinateur permettant d'acquérir des données sensorielles en provenance de dispositifs intelligents environnants, le procédé consistant à :
(a) fournir un dispositif portable intelligent, le dispositif portable intelligent comprenant :
(i) un boîtier, le boîtier renfermant des composants d'un dispositif portable intelligent ;
(ii) un ou plusieurs capteurs configurés pour acquérir des données de capteur, au moins un capteur étant un capteur biologique configuré pour acquérir des données biologiques concernant un utilisateur ;
(iii) une ou plusieurs interfaces de communication ; et
(iv) un processeur ;
(b) diffuser une requête en provenance du dispositif portable intelligent vers des dispositifs intelligents environnants afin de déterminer une disponibilité de dispositifs intelligents environnants accessibles engagés dans l'acquisition de données de capteur qui se rapportent aux données de capteur acquises par le ou les capteurs du dispositif portable intelligent, une pertinence étant basée sur des critères définis ; et
(c) acquérir les données de capteur pertinentes à partir de dispositifs intelligents environnants accessibles, les données de capteur pertinentes servant à remplacer des données de capteur acquises par le ou les capteurs du dispositif portable intelligent qui sont incomplètes ou à corriger des données de capteur incorrectes acquises par le ou les capteurs du dispositif portable intelligent sur le dispositif portable intelligent ;
(d) dans lequel ledit procédé est réalisé en exécutant une programmation sur au moins un processeur informatique, ladite programmation se trouvant sur un support non transitoire lisible par le processeur informatique.

7. Procédé selon la revendication 6, dans lequel l'au moins un parmi le ou les capteurs est un capteur environnemental configuré pour acquérir des données de capteur contextuelles.

8. Procédé selon la revendication 6, consistant en outre à :
envoyer un signal à un ou plusieurs des dispositifs intelligents environnants afin de déclencher une activation ou une désactivation d'une fonction ou de changer la logique pour l'acquisition de données de capteur par les dispositifs portables intelligents supplémentaires.

9. Procédé selon la revendication 6, dans lequel le dispositif portable intelligent possède une plateforme choisie dans le groupe consistant en dispositifs portés à la main, dispositifs portés au doigt, dispositifs portés au poignet, dispositifs portés sur la tête, dispositifs portés au bras, dispositifs portés à la jambe, dispositifs portés à la cheville, dispositifs portés au pied, dispositifs portés aux orteils, montres, lunettes, bagues, bracelets, colliers, articles de bijouterie, articles vestimentaires, chaussures, chapeaux, lentilles de contact et gants.

10. Procédé selon la revendication 6, dans lequel la ou les interfaces de communication sont choisies dans le groupe consistant en une interface de communication câblée, une interface de communication sans fil, une interface de communication cellulaire, une interface de communication Wi-Fi, une interface de communication en champ proche, une interface de communication infrarouge et une interface de communication Bluetooth.

11. Système permettant d'acquérir des données sensorielles en provenance de dispositifs intelligents environnants, le système comprenant :
(a) un ou plusieurs dispositifs intelligents environnants, le ou les dispositifs intelligents comprenant :
(i) un boîtier, le boîtier renfermant des composants d'un dispositif portable intelligent ;
(ii) un ou plusieurs capteurs, au moins un capteur étant configuré pour acquérir des données biologiques ;
(iii) une ou plusieurs interfaces de communication ;
(iv) un processeur ; et
(v) une programmation se trouvant dans un support non transitoire lisible par ordinateur, la programmation étant exécutable par le processeur informatique et configurée pour :
recevoir une requête pour des données de capteur en provenance d'un dispositif portable intelligent ; et
transmettre les données de capteur demandées au dispositif portable intelligent ; et
(b) un dispositif portable intelligent comprenant :
(i) un boîtier, le boîtier renfermant des composants d'un dispositif portable intelligent ;
(ii) un ou plusieurs capteurs configurés pour acquérir des données de capteur, au moins un capteur étant un capteur biologique configuré pour acquérir des données biologiques concernant un utilisateur ;
(iii) une ou plusieurs interfaces de communication ;
(iv) un processeur ; et
(v) une programmation se trouvant dans un support non transitoire lisible par ordinateur, la programmation étant exécutable par le processeur informatique et configurée pour :
diffuser une requête vers les dispositifs intelligents environnants afin de déterminer une disponibilité de dispositifs intelligents environnants accessibles engagés dans l'acquisition de données de capteur qui se rapportent aux données de capteur acquises par le ou les capteurs du dispositif portable intelligent, une pertinence étant basée sur des critères prédéfinis ; et
acquérir les données de capteur pertinentes en provenance des dispositifs intelligents environnants accessibles, les données de capteur pertinentes servant à remplacer des données de capteur acquises par le ou les capteurs du dispositif portable intelligent qui sont incomplètes ou à corriger des données de capteur incorrectes acquises par le ou les capteurs du dispositif portable intelligent sur le dispositif portable intelligent.

12. Système selon la revendication 11, dans lequel au moins un parmi le ou les capteurs de dispositifs intelligents environnants est un capteur environnemental configuré pour acquérir des données de capteur contextuelles, et
au moins un parmi le ou les capteurs du dispositif portable intelligent est un capteur environnemental configuré pour acquérir des données de capteur contextuelles.

13. Système selon la revendication 11, dans lequel ladite programmation sur le dispositif intelligent portable est en outre configurée pour :
envoyer un signal à un ou plusieurs des dispositifs intelligents environnants afin de déclencher une activation ou une désactivation d'une fonction ou de changer la logique pour l'acquisition de données de capteur par les dispositifs portables intelligents supplémentaires.

14. Système selon la revendication 11, dans lequel le dispositif portable intelligent possède une plateforme choisie dans le groupe consistant en dispositifs portés à la main, dispositifs portés au doigt, dispositifs portés au poignet, dispositifs portés sur la tête, dispositifs portés au bras, dispositifs portés à la jambe, dispositifs portés à la cheville, dispositifs portés au pied, dispositifs portés aux orteils, montres, lunettes, bagues, bracelets, colliers, articles de bijouterie, articles vestimentaires, chaussures, chapeaux, lentilles de contact et gants.

15. Système selon la revendication 11, dans lequel la ou les interfaces de communication sur le dispositif portable intelligent sont choisies dans le groupe consistant en une interface de communication câblée, une interface de communication sans fil, une interface de communication cellulaire, une interface de communication Wi-Fi, une interface de communication en champ proche, une interface de communication infrarouge et une interface de communication Bluetooth.
